# EUROPEAN PATENT APPLICATION

(11) **EP 3 831 411 A1**
(43) Date of publication of application: **09.06.2021**
(21) Application number: 19844685.8
(22) Date of filing: 23.07.2019
(51) Int. Cl.: A61K 47/32, A23L 23/10, A23L 33/15, A61K 8/02, A61K 9/20, A61K 47/26, A61K 47/36, A61K 47/38, C11D 3/20

(54) **DISINTEGRATING SOLID CONTAINING DISINTEGRANT COMPONENT AND MICROFIBROUS CELLULOSE**

(30) Priority: 31.07.2018 JP 2018143371
(71) Applicant: Daicel Corporation, Osaka 530-0011 (JP)
(72) Inventor: OKABAYASHI Tomohito, Tokyo 108-8230 (JP); SAKAGUCHI Anan, Tokyo 108-8230 (JP)
(74) Representative: J A Kemp LLP
(86) International application number: PCT/JP2019/028758
(87) International publication number: WO 2020/026881

(57) **Abstract**

To provide a technique for accelerating disintegration without using a foaming agent, an acidic component, and the like in a solid (such as a tablet) that needs to be dissolved in a liquid when used.

[Solution]

To provide: a disintegrating particulate composition containing microfibrous cellulose; a disintegrating solid containing the composition; and the like.

## Description

### Technical Field

The present invention relates to a disintegrating solid and the like containing a disintegrant component and microfibrous cellulose, the disintegrating solid and the like being relatively large in shape and weight.

### Background Art

In the related art, regarding a solid (such as a tablet) that needs to be dissolved in a liquid when used, such as a cleaning agent, a disinfectant, an insecticide, a seasoning processed food, a solid seasoning, a powdered beverage, an bathing agent, a gargling agent, a water conditioning agent, a mineral reinforcing agent, and the like, in order to accelerate the disintegration of the solid, common additives such as sugar, sugar alcohols, and starches, foaming agents such as carbonate, and acidic components such as citric acid, ascorbic acid, succinic acid, fumaric acid, and malic acid have been used. Alternatively, a special method such as freeze-drying was used to reduce the tablet density and accelerate disintegration.

Patent Document 1 discloses: a disintegrating particulate composition containing a disintegrant component and microfibrous cellulose; and a disintegrant containing the composition.

Cellulose that is produced from plant fibers and that has a fiber diameter (short diameter) or thickness of from approximately several nm to several µm has been generally known as "fine fibrous cellulose" or "microfibrous cellulose". The production examples of such microfibrous cellulose; and a structure, properties, and functions thereof are disclosed in Patent Documents 2 and 3.

In the fine fibrous or microfibrous cellulose, a surface area is remarkably increased and hydrophilicity, which is an intrinsic characteristic of cellulose, is remarkably strengthened, and a three-dimensional network structure is formed through entanglement of microfibers, without deteriorating basic properties (such as physical and chemical stabilities, crystal form (I type)) of cellulose of a raw material. As a result, when it is formulated into goods in a paste or cream shape, it exerts a water-retaining (water separation prevention) and form-retaining effects due to the interaction with water, oil droplets, fine particles, and the like. Further, it is also utilized for modification such as improving the strength of jelly-like goods due to the three-dimensional network structure.

Thus, these celluloses have been widely used in various applications, for example, as a binder for various powder and fibrous materials, a paper strengthening agent in sandpaper, a thickening agent for improving food texture of foods, a humectant for water-retaining of foods, a filter aid for alcoholic beverages, and the like.

As an application example of the fine fibrous cellulose, Patent Document 4 discloses a jelly composition containing: a water-dispersible complex containing, in a particular ratio, the fine fibrous cellulose and a hydrophilic polymer that is soluble in warm water; a gelling agent; and water in a particular ratio. It is disclosed that the composition has properties to inhibit denaturation of proteins and precipitation of water-insoluble components during heating or warming treatment and to give a good texture.

Additionally, Patent Document 5 discloses a gelling agent containing: a highly dispersible cellulose complex containing fine fibrous cellulose, a water-soluble polymer, and hydrophilic substance in a particular ratio; and a particular kind of polysaccharide in a particular ratio. It is disclosed that the agent is superior in disintegrability and dispersibility in water when compared to a highly dispersible cellulose complex in the related art, and thus can be used in industrial and practical dispersing conditions.

Thus, in the invention disclosed in any of Patent Documents 4 or 5, the fine fibrous cellulose is used as a one component in the gelly composition and gelling agent. Furthermore, the hydrophilic polymer is an essential component for the water-dispersible complex disclosed in Patent Document 3, and the water-soluble polymer is an essential component for the highly dispersible cellulose complex disclosed in Patent Document 4.

Further, Patent Document 6 discloses an orally disintegrating tablet that is a tablet having a large weight (diameter: 12 to 18 mm, weight: approximately 15 to 250 mg) and being relatively thin and that has an extremely short oral disintegration time and/or water disintegration time. However, the orally disintegrating tablet disclosed in examples of the aforementioned document does not contain microfibrous cellulose.

### Citation List

### Patent Document

Patent Document 1: WO 2015/163135
Patent Document 2: JP 56-100801 A
Patent Document 3: JP 2009-203559 A
Patent Document 4: JP 2004-283135 A
Patent Document 5: JP 2006-290972 A
Patent Document 6: WO 2016/073917

### Summary of Invention

### Technical Problem

In the related art, since a foaming agent and an acidic component are contained in a solid and thus accelerate the disintegration of the solid as described above, it is necessary to take into account: the packaging and internal state of the solid; and a change in pH with respect to a system containing the solid. For example, there have been various disadvantages such as: expansion or rupture of a packaging due to generation of carbon dioxide gas derived from the foaming agent in the packaging, difficulty in application of an acidicly unstable active ingredient, denaturation of various components due to a change in pH of the liquid (for example, soup stock in a pot) into which the solid is added, and a decrease in the activity of the detergent.

Therefore, there has been a demand for techniques for accelerating the disintegration of the solid as described above without using foaming agents, acidic components, and the like.

An object of the present invention is to solve this problem and is to provide a disintegrating solid containing microfibrous cellulose and the like.

### Solution to Problem

To solve the above problems, the present inventors have diligently studied to find an unexpected effect that, by adding microfibrous cellulose to a known composition in the related art having an application as a disintegrant, due to the synergistic effect of both, the tablet hardness in various disintegrating tablets containing the composition is increased, while water disintegration time is shortened. With this, the present invention was arrived at.

The present invention provides the following aspects.

[Aspect 1] A disintegrating solid containing: a disintegrant component and microfibrous cellulose.
[Aspect 2] The disintegrating solid according to aspect 1, wherein a density is from 0.80 to 3.00 mg/mm³ and a weight is 1000 mg or more.
[Aspect 3] A rapidly disintegrating tablet according to aspect 1 or 2, wherein a diameter is 14 mm or more.
[Aspect 4] The disintegrating solid according to aspects 1 to 3, wherein an absolute hardness of a tablet is from 0.01 N/mm² to 4.00 N/mm².
[Aspect 5] The disintegrating solid according to any one of aspects 1 to 4, wherein water disintegration time is 4 minutes or less.
[Aspect 6] The disintegrating solid according to aspect 1, wherein the microfibrous cellulose has an average fiber length from 0.01 to 2 mm and an average fiber diameter from 0.001 to 1 µm.
[Aspect 7] The disintegrating solid according to any one of aspects 1 to 6, wherein the disintegrant component is one component or more selected from crospovidone, sodium croscarmellose, low degree of substitution hydroxypropyl cellulose, carboxymethyl cellulose calcium, starch, and modified starch.
[Aspect 8] The disintegrating solid according to aspect 7, wherein the starch is corn starch, potato starch, waxy corn starch, partially α-starch, or α-starch, and modified starch is sodium starch glycolate or hydroxypropyl starch.
[Aspect 9] The disintegrating solid according to any one of aspects 1 to 6 further containing sugar alcohol or sugar.
[Aspect 10] The disintegrating solid according to any one of aspects 1 to 6 further containing an auxiliary excipient.
[Aspect 11] The disintegrating solid according to aspect 10, wherein the auxiliary excipient is crystalline cellulose and/or powdered cellulose.
[Aspect 12] A method for production of the disintegrating solid according to any one of aspects 1 to 11, the method including: mixing a disintegrating particulate composition containing a disintegrant component and microfibrous cellulose with an active ingredient; and tableting an obtained mixture.
[Aspect 13] The method for production according to aspect 12 further including tableting by applying a tableting compressive force of from 10 to 100 kN.

### Advantageous Effects of Invention

Regardless of relatively large shape and weight of the disintegrating solid according to an embodiment of the present invention, by containing the disintegrant component and the microfibrous cellulose, the excellent disintegrability required for various disintegrating solids can be imparted by the synergistic effect thereof.

Due to the excellent disintegrability of the disintegrating solid according to an embodiment of the present invention, for example, the dispersibility of the active ingredient contained in the solid such as a cleaning agent is improved, and thus the time until the effect is exhibited can be shortened. In a case where the active ingredient is a seasoning, the disintegration is performed quickly, which has an advantage of shortening a cooking time. In addition, in a case where the disintegrating solid is a powdered beverage, the disintegration is performed rapidly, and the beverage can be obtained in an extremely short time. Furthermore, in the disintegrating solid according to an embodiment of the present invention, unlike the related art, since it is not necessary to contain: a foaming agent such as carbonate; and an acidic component to accelerate the disintegration of the solid, changes in the pH of the active ingredient, accompanying changes in taste, and decreased activity of the active ingredient are unlikely to occur.

### Brief Description of Drawings

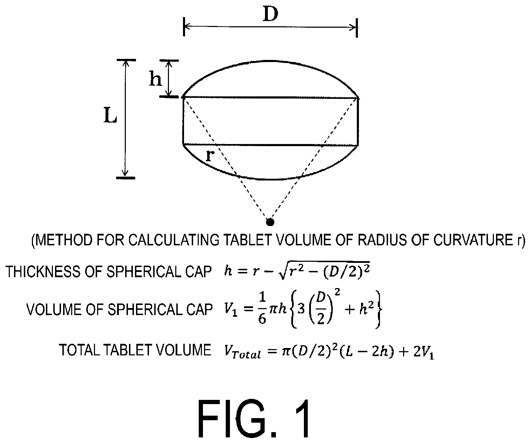

FIG. 1 illustrates a method for calculating a volume of a disintegrating solid (a tablet with an R surface) according to an embodiment of the present invention.

### Description of Embodiments

The present invention relates to a disintegrating solid containing a disintegrant component and microfibrous cellulose. As the microfibrous cellulose, any cellulose known as "fine fibrous cellulose" or "microfibrous cellulose" known in the related art can be used.

The disintegrating solid according to an embodiment of the present invention is not particularly administered and ingested directly into the body like an orally disintegrating tablet and besides, has applications to those that need to be dissolved in a liquid when used, such as a cleaning agent, a disinfectant, an insecticide, a seasoning processed food, a solid seasoning, a powdered beverage, an bathing agent, a gargling agent, a water conditioning agent, a mineral reinforcing agent, and the like.

As described above, "microfibrous cellulose" means cellulose that is generally produced from plant fibers and that has a fiber diameter (short diameter) or thickness from several nm to several µm, wherein the surface area is remarkably increased and hydrophilicity, which is an intrinsic characteristic of cellulose, is remarkably strengthened, and a three-dimensional network structure is formed through entanglement of microfibers, without deteriorating basic properties (such as physical and chemical stabilities and the like) of cellulose of a raw material.

A crystal structure of cellulose can be broadly classified into type I to type IV The cellulose type I has only natural cellulose. The cellulose produced by living tissue is an aggregate of single crystal fibers, and all cellulose molecular chains in the single crystal fibers have identical parallel chains and are oriented in the same direction. The cellulose type II is obtained by treating natural cellulose fibers with a concentrated alkaline solution, washing with water, and drying. A filling mode of the molecules is antiparallel chains, and the adjacent molecular chains are oriented in opposite directions. The cellulose type III is obtained by treating natural cellulose fiber or the cellulose type II with liquid ammonia, washing, and drying. The cellulose type IV is obtained by heating the natural cellulose fiber or the cellulose type II to 280°C in glycerol. There is no retransformation from cellulose types II, III and IV to the natural cellulose fiber type I, and this process is an irreversible process. The microfibrous cellulose used in an embodiment of the present invention preferably has a crystalline structure of the cellulose type I.

The dried product of such microfibrous cellulose can be obtained by any known technique in the related art, including for example, directly pulverizing cellulose fibers in a dry state using a ball mill to directly obtain the microfibrous cellulose in a dry state (Patent Document 2). Alternatively, in a replacement step, microfibrous cellulose in an aqueous suspension state, composed of microfibrous cellulose obtained by microfibrillating an aqueous dispersion liquid of cellulose fibers using a high pressure homogenizer, is subjected to solvent replacement, after which, in a drying step, the solvent is removed, and in a pulverizing step, the material is then pulverized, thereby a dried product of microfibrous cellulose can be obtained (Patent Document 3).

Preferred examples of the microfibrous cellulose contained in the disintegrating particulate composition according to an embodiment of the present invention include microfibrous cellulose that is a fiber aggregate having: an average fiber length of approximately from 0.01 to 2 mm; and an average fiber diameter of approximately from 0.001 to 1 µm and preferably approximately from 0.01 to 0.1 µm (Patent Document 2). For example, various grades of products of such microfibrous cellulose (water-containing state with solid content from 10 to 35%) are commercially available from Daicel FineChem Ltd. under the series trade name "Celish" (average fiber diameter of approximately from 0.01 to 0.1 µm).

Any material known to those skilled in the art can be used as the disintegrant component contained in the disintegrating solid according to an embodiment of the present invention. For example, it can contain any one or more components selected from: starches such as crospovidone, sodium croscarmellose, low degree of substitution hydroxypropyl cellulose, carboxymethyl cellulose calcium, corn starch, potato starch, waxy corn starch, partially α-starch, and α-starch; and modified starches such as sodium starch glycolate and hydroxypropyl starch. Note that, crospovidone is a common name for a crosslinked polymer of 1-vinyl-2-pyrrolidone, and sodium croscarmellose is a common name for a crosslinked product of sodium carboxymethyl cellulose.

Note that among such disintegrant components, for example, crospovidone, sodium croscarmellose, low degree of substitution hydroxypropyl cellulose, and carboxymethyl cellulose calcium are all water-insoluble polymers. Further, as starch, for example, corn starch, potato starch, waxy corn starch, water-insoluble partially α-starch, and water-insoluble α-starch are water-insoluble polymers, and as the modified starch, for example, modified starch such as sodium starch glycolate and hydroxypropyl starch is a water-insoluble polymer.

The disintegrating solid according to an embodiment of the present invention may further contain any sugar alcohol or sugar known to those skilled in the art as an excipient.

Representative examples of sugar alcohol or sugar include mannitol, erythritol, xylitol, trehalose, lactose, maltose, maltitol, glucose, sucrose, fructose, mannose, and sorbitol. Further, preferred examples include mannitol, erythritol, xylitol, trehalose, and lactose. In addition, one type of sugar or sugar alcohol may be used, and two or more types of compounds appropriately selected from these may also be used.

Furthermore, by containing crystalline cellulose and/or powdered cellulose as an auxiliary excipient in the disintegrating solid according to an embodiment of the present invention, it is possible to impart superior tablet hardness and disintegrability to the disintegrating tablet. The crystalline cellulose and powdered cellulose are white powder substances that are insoluble in water and that are obtained by partially depolymerizing and purifying α-cellulose obtained from a fibrous plant with acid. Since they have no taste and are chemically inert, there is no change when mixed with drugs, and they are used in applications such as pharmaceutical additives, particularly auxiliary excipients, binders or disintegrants when preparing tablets. Further, other than pharmaceuticals, it is used as an emulsion stabilizer and the like in cosmetics and foods such as dairy products.

Any crystalline cellulose and/or powdered cellulose known to those skilled in the art can be used, and representative examples of commercially available crystalline cellulose products include Avicel (FMC Corporation), Ceolus (Asahi Kasei Chemicals Corp.), and Vivapur (Rettenmaier Japan Co., Ltd.), and representative examples of powdered cellulose can include KC Flock (Nippon Paper Chemicals Co., Ltd.), ARBOCEL (Rettenmaier Japan Co., Ltd.), and Solka Floc (Kimura Sangyo Co., Ltd.).

Depending on the purpose and application of the solid according to an embodiment of the present invention, various types of active ingredients (active constituents) can be included in any amount.

Examples of such active ingredients include any of various components known to those skilled in the art and contained in, for example, a cleaning agent, a disinfectant, an insecticide, a seasoning processed food, a solid seasoning, a powdered beverage, an bathing agent, a gargling agent, a water conditioning agent, a mineral reinforcing agent, and the like, which are main applications of the solid according to an embodiment of the present invention described above.

Furthermore, various optional components known to those skilled in the art may be appropriately added and mixed in the disintegrating solid according to an embodiment of the present invention, for example, for the purpose of adjusting various characteristics such as disintegration force, bonding force, and feeling of taking tablets, and the like, within a range that does not impair the effects of the present invention. Examples of such components include an excipient, a disintegrant, an auxiliary excipient, a fluidizing agent, a lubricant, a sweetener, a flavoring agent, a flavor, a colorant, and the like known to those skilled in the art.

The compounded amount of each component in the disintegrating solid according to an embodiment of the present invention can be appropriately determined by those skilled in the art depending on the solid component and the type and application of each component. Generally, based on total weight of the disintegrating solid, the microfibrous cellulose (in terms of dried product) is in the range of from 1 wt.% to 50 wt.%, the disintegrant component is in the range of from 1 wt.% to 30 wt.%, the sugar or sugar alcohol (if included) is in the range of from 20 wt.% to 98 wt.%, and the auxiliary excipient (if included), such as crystalline cellulose and/or powdered cellulose is in the range of from 1 wt.% to 40 wt.%.

The disintegrating solid according to an embodiment of the present invention has a relatively large shape (size) and weight, for example, as compared to pharmaceutical tablets and food tablets that are ingested directly into the body. Note that the form and structure of the solid is not particularly limited and may take any form or structure known to those skilled in the art such as tablets, sheets, capsules, granules, and the like.

The disintegrating solid according to an embodiment of the present invention is relatively large in shape and weight. That is, the weight is usually 1000 mg or more and preferably 1500 mg or more. Further, the density thereof is usually in the range of from 0.80 to 3.00 mg/mm³ and preferably from 1.1 to 2.00 mg/mm³.

When the disintegrating solid according to an embodiment of the present invention is a tablet, any shape known to those skilled in the art (for example, a straight tablet, a standard R surface, a sugar-coated R surface, a round-corner flat tablet (flat surface), an angled-corner flat tablet (flat surface), and two-stage R surface) can be taken. In addition, the diameter of the tablet can be set as appropriate, and the diameter is usually 14 mm or more and preferably 20 mm or more, and the thickness is usually 4 mm or more and preferably 6 mm or more.

Further, the disintegrating solid according to an embodiment of the present invention have excellent tablet hardness and disintegrability. For example, when tableting is performed by applying a tableting compressive force of from 10 to 100 kN, the absolute hardness of the disintegrating solid is from 0.01 N/mm² to 4.00 N/mm², preferably from 0.05 N/mm² to 2.50 N/mm², and more preferably from 0.10 N/mm² to 2.50 N/mm², and the water disintegration time is 4 minutes or less, preferably 3 minutes or less, and particularly preferably 1 minute or less.

The disintegrating solid according to an embodiment of the present invention can be made by any method or means known to those skilled in the art. For example, a disintegrating particulate composition containing a disintegrant component and microfibrous cellulose can be mixed with the active ingredient, and the obtained mixture is produced by, for example, a production method including tableting the obtained mixture by applying the tableting compressive force of from 10 to 100 kN.

The disintegrating particulate composition according to an embodiment of the present invention can be made by any method known to those skilled in the art.

For example, the disintegrating particulate composition described above can be produced by mixing various components contained in the composition at once.

Alternatively, it can be produced by various granulation methods. Granulation means is not particularly limited and a dry granulation method, a wet granulation method, or the like can be used to produce the composition.

The dry granulation method includes: mixing the various component powders contained in the disintegrating particulate composition as is or with an appropriate binder and the like; breaking the resulting mixture into small bulks with a high pressure; and appropriately crushing and granulating the powder. Specific examples of the dry granulation method include a crushing granulation method and a roller compaction method.

The wet granulation method is a method of forming a complex by dispersing and drying each ingredient in the presence of water, and specific examples of wet granulation method include: spray methods such as spray drying, rolling granulation, agitation granulation, and fluidized bed granulation; freeze drying method; and kneading granulation. The easy-to-take granular preparation and granules according to an embodiment of the present invention can be produced by any of these methods known in the art.

When produced in a wet granulation method, the disintegrating particulate composition according to an embodiment of the present invention may be produced in a one-stage granulation step in which all of the components contained in the disintegrating particulate composition are used together or may be added and mixed in a multiple stage wet granulation step.

For example, it can be produced by: a first wet granulation step of using any one or two components; a two-stage granulation step including a second wet granulation step using at least the granulated matter obtained in the first wet granulation step and the remaining components; or a three-stage granulation step including a third step of further mixing the components with the granulated matter obtained in the second wet granulation step.

Note that, in a plurality of wet granulation steps of the production method described above, the use of any one or two types of components among the components contained in the disintegrating particulate composition can be appropriately determined by those skilled in the art depending on the types, amount, and the like.

Furthermore, in each granulation step, various conditions such as spray rate, air feed air temperature, exhaust temperature, and air supply amount can be appropriately determined by those skilled in the art depending on the type, amount, and the like of each component.

In each granulation step, as a medium for the spray liquid, a solvent acceptable in a pharmaceuticals or foods, such as water, ethanol, methanol, or acetone can be mentioned. Alternatively, as the spray liquid, an aqueous solution in which less than 10% of component of the disintegrating particulate composition is dissolved can be mentioned, and water or such an aqueous solution is particularly preferable.

For example, the disintegrating particulate composition according to an embodiment of the present invention can be produced by spraying a dispersion liquid (slurry) of microfibrous cellulose onto a fluidized bed granulator containing all the components except for the microfibrous cellulose.

Note that, the various optional components described above that can be appropriately contained in the disintegrating particulate composition according to an embodiment of the present invention can be appropriately added in each granulation step. Alternatively, the optional components can be added and mixed in a stage in an additionally provided wet granulation step.

It is preferable that the disintegrating particulate composition according to an embodiment of the present invention produced by such a wet granulation method has the following physical properties.
(1) Average particle size: from 50 to 200 microns; (2) Moisture: from 0.5 wt.% to 7 wt.%.

Note that, these physical property values are measured according to the following conditions and methods.

Average particle size: 2 g of disintegrating particulate composition is measured using an ϕ75 mm automatic shaking sieve (M-3T type, Tsutsui Scientific Instruments Co., Ltd).

Moisture: 5 g of the disintegrating particulate composition is measured using a halogen moisture meter (MX-50, A&D Company, Limited).

Note that all of the details described in the prior art documents cited in the present specification are incorporated herein as references.

Hereinafter, the present invention is more specifically described through examples, but the present invention is not limited by these examples.

### Example 1

### [Production of disintegrating particulate composition 1]

270 g of lactose monohydrate (GranuLac, Meggle Japan Co., Ltd.), 80 g of corn starch (Cornstarch, Nihon Shokuhin Kako Co., Ltd.), and 20 g of partially α-starch (PCS PC-10, Asahi Kasei Chemicals Co., Ltd.) were added to a fluidized bed granulator (FL-LABO, Freund corp.), a wet body of microfibrous cellulose (CELISH FD200L, Daicel Fine Chem Ltd.) was diluted with water to make 5%, 600 g of a CELISH suspension was sprayed at a rate of 12 g/min to granulate, and thereby a disintegrating particulate composition 1 was obtained. Note that, the obtained disintegrating particulate composition 1 has the following physical properties. (1) Average particle size: 135 microns; (2) Moisture: 2.5 wt.%.

### [Production of disintegrating solid 1]

0.5 parts by weight of calcium stearate (Taihei Chemical Industrial Co., Ltd.) was added to 99.5 parts by weight of the obtained disintegrating particulate composition 1 and was mixed with each other. The mixture was then subjected to tableting at a tableting compressive force of 25 kN with a simple tableting machine (HANDTAB-100, ICHIHASHI SEIKI Co., Ltd.) to obtain an angled-corner flat tablet having a diameter of 14.0 mm, a weight of 1500 mg, and a thickness of 7.4 mm.

### Example 2

### [Production of disintegrating solid 2]

The obtained disintegrating particulate composition 1 was tableted in the same manner as Example 1 to obtain an angled-corner flat tablet having a diameter of 20.0 mm, a weight of 2500 mg, and a thickness of 6.8 mm.

### Example 3

### [Production of disintegrating solid 3]

0.5 parts by weight of calcium stearate (Taihei Chemical Industrial Co., Ltd.) was added to: 50 parts by weight of soup stock (powder, SHIMAYA Co., Ltd.) as an active ingredient; and 49.5 parts by weight of the obtained disintegrating particulate composition 1 and was mixed with each other. The mixture was then subjected to tableting at a tableting compressive force of 15 kN with a simple tableting machine (HANDTAB-100, ICHIHASHI SEIKI Co., Ltd.) to obtain an angled-corner flat tablet having a diameter of 20.0 mm, a weight of 2500 mg, and a thickness of 7.4 mm.

### Example 4

### [Production of disintegrating solid 4]

0.5 parts by weight of calcium stearate (Taihei Chemical Industrial Co., Ltd.) was added to: 50 parts by weight of soup stock (powder, SHIMAYA Co., Ltd.) as an active ingredient; and 49.5 parts by weight of the obtained disintegrating particulate composition 1 and was mixed with each other. The mixture was then subjected to tableting at a tableting compressive force of 25 kN with a simple tableting machine (HANDTAB-100, ICHIHASHI SEIKI Co., Ltd.) to obtain an angled-corner flat tablet having a diameter of 20.0 mm, a weight of 2500 mg, and a thickness of 6.3 mm.

### Example 5

### [Production of disintegrating solid 5]

0.5 parts by weight of calcium stearate (Taihei Chemical Industrial Co., Ltd.) was added to: 50 parts by weight of ascorbic acid (vitamin C, Mitsubishi-Chemical Foods Corporation) as an active ingredient; and 49.5 parts by weight of the obtained disintegrating particulate composition 1 and was mixed with each other. The mixture was then subjected to tableting at a tableting compressive force of 25 kN with a simple tableting machine (HANDTAB-100, ICHIHASHI SEIKI Co., Ltd.) to obtain an angled-corner flat tablet having a diameter of 20.0 mm, a weight of 2500 mg, and a thickness of 6.8 mm.

### Example 6

### [Production of disintegrating solid 6]

0.5 parts by weight of calcium stearate (Taihei Chemical Industrial Co., Ltd.) was added to: 50 parts by weight of sodium chloride (Wako Pure Chemical Industries, Ltd.) as an active ingredient; and 49.5 parts by weight of the obtained disintegrating particulate composition 1 and was mixed with each other. The mixture was then subjected to tableting at a tableting compressive force of 25 kN with a simple tableting machine (HANDTAB-100, ICHIHASHI SEIKI Co., Ltd.) to obtain an angled-corner flat tablet having a diameter of 20.0 mm, a weight of 2500 mg, and a thickness of 5.7 mm.

### Comparative Example 1

300 g of lactose monohydrate (GranuLac, Meggle Japan Co., Ltd.), 80 g of corn starch (Cornstarch, Nihon Shokuhin Kako Co., Ltd.), and 20 g of partially α-starch (PCS PC-10, Asahi Kasei Chemicals Co., Ltd.) were added to obtain a mixture 1.

The obtained mixture 1 was tableted in the same manner as Example 4 to obtain an angled-corner flat tablet having a diameter of 20.0 mm, a weight of 2500 mg, and a thickness of 7.5 mm.

### [Evaluation of hardness and disintegrability test]

Hardness (absolute hardness) and water disintegration time were measured for each of the tablets obtained in the above Examples and Comparative Examples by the following method. Table 1 indicates the measurement results of hardness and disintegration time.

Note that, these physical property values were measured according to the following conditions and methods.

Density is "tablet weight/tablet volume". The density can be determined in a common manner that is generally performed. For example, in a case of a flat tablet, assuming that it is a cylinder, a tablet volume can be obtained from a bottom area and a tablet thickness of the tablet to calculate the density, and in a case of a cube tablet, assuming that it is a cube or a rectangular parallelepiped, and the tablet volume can be obtained to calculate the density. In addition, in a case of a tablet having an R surface, the tablet volume is obtained by the method of FIG. 1, and the density can be calculated.

Hardness: Hardness (N) was measured using a digital wooden hardness meter (FUJIWARA SCIENTIFIC CO., LTD).

Absolute hardness is hardness which does not depend on the thickness of the tablet or the diameter of the tablet. The hardness (N) obtained by a digital wooden hardness meter can be calculated. For example, in the case of a flat tablet, the absolute hardness can be calculated from absolute hardness (N/mm2) = (2 × hardness (N))/ (π × tablet diameter (mm) × tablet thickness (mm)).

Water disintegration time: The water disintegration time was measured using a disintegration tester (NT-400, Toyama Sangyo Co., Ltd.) in a method using an auxiliary cylinder according to the Japanese Pharmacopeia description (but without the auxiliary plate).

The hardness (and absolute hardness) and the disintegration time were measured six times each, and the average value thereof was used as the measurement result.

**[Table 1]**

| Disintegrating solid | Example 1 | Example 2 | Example 3 |
|---|---|---|---|
| Tableting compressive force (kN) | 25 | 25 | 15 |
| Density (mg/mm³) | 1.31 | 1.17 | 1.32 |
| Hardness (N) | 295 | 157 | 14 |
| Absolute hardness (N/mm²) | 1.80 | 0.74 | 0.20 |
| Water disintegration time (sec) | 50 | 34 | 38 |

**[Table 2]**

| Disintegrating solid | Example 4 | Example 5 | Example 6 | Comparative Example 1 |
|---|---|---|---|---|
| Tableting compressive force (kN) | 25 | 25 | 25 | 25 |
| Density (mg/mm³) | 1.27 | 1.17 | 1.39 | 1.07 |
| Hardness (N) | 31 | 24 | 42 | 12 |
| Absolute hardness (N/mm²) | 0.16 | 0.11 | 0.23 | 0.05 |
| Water disintegration time (sec) | 34 | 53 | 39 | 290 |

From the results indicated in Table 1, in Examples 1 to 6, as compared to the solid (Comparative Example 1) obtained by using the composition containing no microfibrous cellulose, the disintegrating solid obtained by using the microfibrous cellulose has more excellent disintegrability and moldability, and it was demonstrated to show a rapid disintegration time without containing a foaming agent such as carbonate as an additive used for the purpose of promoting disintegration.

### Example 7

### [Production of disintegrating solid 7]

0.5 parts by weight of calcium stearate (Taihei Chemical Industrial Co., Ltd.) was added to: 50 parts by weight of sodium sulfate (main component of bathing agent) as an active ingredient; and 49.5 parts by weight of the obtained disintegrating particulate composition 1 and was mixed with each other. The mixture was then subjected to tableting at a tableting compressive force of 15 kN with a simple tableting machine (HANDTAB-100, ICHIHASHI SEIKI Co., Ltd.) to obtain an angled-corner flat tablet having a diameter of 20.0 mm, a weight of 2500 mg, and a thickness of 5.4 mm.

### Example 8

### [Production of disintegrating solid 8]

0.5 parts by weight of calcium stearate (Taihei Chemical Industrial Co., Ltd.) was added to: 50 parts by weight of sodium bicarbonate (main component of cleaning agent) as an active ingredient; and 49.5 parts by weight of the obtained disintegrating particulate composition 1 and was mixed with each other. The mixture was then subjected to tableting at a tableting compressive force of 15 kN with a simple tableting machine (HANDTAB-100, ICHIHASHI SEIKI Co., Ltd.) to obtain an angled-corner flat tablet having a diameter of 20.0 mm, a weight of 2500 mg, and a thickness of 6.2 mm.

The results evaluated using the same manner as Examples 1 to 6 are indicated in Table 3.

It was demonstrated that even when an active ingredient commonly used in the bathing agent and cleaning agent was used, a rapid disintegration time was exhibited.

**[Table 3]**

| Disintegrating solid | Example 7 | Example 8 |
|---|---|---|
| Tableting compressive force (kN) | 15 | 15 |
| Density (mg/mm³) | 1.47 | 1.28 |
| Hardness (N) | 27 | 25 |
| Absolute hardness (N/mm²) | 0.16 | 0.13 |
| Water disintegration time (sec) | 37 | 38 |

### Industrial Applicability

The present invention greatly contributes to research and development of various disintegrating solids with excellent tablet hardness and disintegrability.

## Claims

1. A disintegrating solid comprising:
a disintegrant component; and
microfibrous cellulose.

2. The disintegrating solid according to claim 1, wherein a density is from 0.80 to 3.00 mg/mm³ and a weight is 1000 mg or more.

3. A rapidly disintegrating tablet according to claim 1 or 2, wherein a diameter is 14 mm or more.

4. The disintegrating solid according to claims 1 to 3, wherein an absolute hardness of a tablet is from 0.01 N/mm² to 4.00 N/mm².

5. The disintegrating solid according to any one of claims 1 to 4, wherein water disintegration time is 5 minutes or less.

6. The disintegrating solid according to claim 1, wherein the microfibrous cellulose has an average fiber length from 0.01 to 2 mm and an average fiber diameter from 0.001 to 1 µm.

7. The disintegrating solid according to any one of claims 1 to 6, wherein the disintegrant component is one component or more selected from crospovidone, sodium croscarmellose, low degree of substitution hydroxypropyl cellulose, carboxymethyl cellulose calcium, starch, and modified starch.

8. The disintegrating solid according to claim 7, wherein the starch is corn starch, potato starch, waxy corn starch, partially α-starch, or α-starch, and modified starch is sodium starch glycolate or hydroxypropyl starch.

9. The disintegrating solid according to any one of claims 1 to 6 further comprising sugar alcohol or sugar.

10. The disintegrating solid according to any one of claims 1 to 6 further comprising an auxiliary excipient.

11. The disintegrating solid according to claim 10, wherein the auxiliary excipient is crystalline cellulose and/or powdered cellulose.

12. A method for production of the disintegrating solid described in any one of claims 1 to 11, the method comprising:
mixing a disintegrating particulate composition containing a disintegrant component and microfibrous cellulose with an active ingredient; and
tableting an obtained mixture.

13. The method for production according to claim 12 further comprising tableting by applying a tableting compressive force of from 10 to 100 kN.
